# EUROPÄISCHE PATENTSCHRIFT

(11) **EP 2 861 168 B1**
(45) Veröffentlichungstag und Bekanntmachung des Hinweises auf die Patenterteilung: **28.06.2017**
(21) Anmeldenummer: 13711570.5
(22) Anmeldetag: 13.03.2013
(51) Int. Cl.: A61B 17/68, A61B 17/80

(54) **SPANNELEMENT ZUM FIXIEREN VON BRUCHENDEN VON KNOCHEN EINER KNOCHENFRAKTUR**
TENSIONING ELEMENT FOR FIXING THE FRACTURE ENDS OF THE BONES IN A BONE FRACTURE
ÉLÉMENT DE SERRAGE POUR FIXER DES EXTRÉMITÉS D'OS FRACTURÉES D'UNE FRACTURE OSSEUSE

(30) Priorität: 13.06.2012 DE 102012105125
(43) Veröffentlichungstag der Anmeldung: 22.04.2015
(73) Patentinhaber: Hipp Medical AG, 78600 Kolbingen (DE)
(72) Erfinder: WAIZENEGGER, Markus, 78600 Kolbingen (DE)
(74) Vertreter: Kuhnen & Wacker
(86) Internationale Anmeldenummer: PCT/EP2013/000749
(87) Internationale Veröffentlichungsnummer: WO 2013/185863

(56) Entgegenhaltungen:
- WO-A2-2005/086708
- DE-U1-202011 109 808
- US-A1- 2005 171 539
- US-A1- 2006 058 796
- US-A1- 2007 299 448
- US-A1- 2008 161 861
- US-A1- 2009 036 930

## Beschreibung

Die Erfindung betrifft ein Spannelement zum Fixieren von Bruchenden von Knochen einer Knochenfraktur nach Anspruch 1.

Bei unzureichender Ruhigstellung einer Knochenfraktur kann eine Kallusbildung, d.h. eine schwielenartige Verdickung der Bruchenden aus überwachsendem Knochengewebe, auftreten. Um eine solche indirekte Frakturheilung über einen Kallus zu vermeiden, werden Knochenplatten verwendet, die auf der Außenfläche eines gebrochenen Knochens aufgebracht und befestigt werden, damit die Bruchstelle während des Heilungsprozesses fixiert ist.

Zudem wird der Heilungsprozess einer Fraktur günstig beeinflusst, wenn eine Kompression auf die zusammengefügte Bruchstelle aufgebracht wird. Hierdurch wird eine besonders enge Adaption, d.h. ein geringes Spaltmaß, über welches die Bruchenden wieder aufeinander zu wachsen, bewirkt.

Aus der DE 20 2011 109808 U1 ist ein Spannelement bekannt, das aus einem durch eine umlaufende Wandung ausgebildeten Konturkörper mit federelastischen Beugeabschnitten und Aufnahmeabschnitten besteht. Die Positionierung des Spannelements erfolgt über die Aufnahmeabschnitte, durch welche Corticalisschrauben hindurchgesteckt werden und in den Knochen eingebracht werden können. Um die gewünschte Kompression der Bruchenden zu erreichen, wird vor der Positionierung bzw. Befestigung des Spannelements am Knochen auf die Beugeabschnitte von Außen ein nach innen gerichteter Druck aufgebracht. Nachteilig stellte sich hingegen bei diesem Spannelement heraus, dass insbesondere in den Beugeabschnitten mechanische Maximalspannungen vorliegen, die zu einem Werkstoffversagen oder eventueller Unförmigkeit des Spannelements führen können.

Dementsprechend ist es die der vorliegenden Erfindung zugrundeliegende Aufgabe, ein Spannelement zu schaffen, mit dem mechanische Maximalspannungen innerhalb des Konturkörpers gleichmäßig verteilt werden können.

Diese Aufgabe wird durch ein Spannelement mit den Merkmalen des Anspruchs 1 gelöst.

Erfindungsgemäß wird ein Spannelement zum Fixieren von Bruchenden von Knochen einer Knochenfraktur mit einem in Draufsicht hohlen, eine umlaufende Wandung aufweisenden Konturkörper vorgeschlagen. Der Konturkörper weist zwei einander gegenüberliegende stirnseitige Aufnahmeabschnitte zur Aufnahme von Befestigungsmitteln, die durch den Konturkörper hindurchsteckbar sind, und zwei Seitenflanken mit Beugeabschnitten auf. Die umlaufende Wandung des Konturkörpers ist wenigstens abschnittsweise im Bereich der stirnseitigen Aufnahmeabschnitte und im Bereich der seitlichen Beugeabschnitte federelastisch verformbar. Ferner ist über die Beugeabschnitte ein definiertes elastisches Verhalten des Spannelements derart eingeprägt, dass eine vorbestimmte Spannung zwischen den Aufnahmeabschnitten erzeugbar ist. Erfindungsgemäß weist der Konturkörper wenigstens ein Gelenk zum Ausgleich und Verteilen von Spannungsspitzen auf, wobei das wenigstens eine Gelenk im Bereich der umlaufenden Wandung vorliegt.

Durch Ausbilden eines Gelenks innerhalb der umlaufenden Wandung können besonders gut die auftretenden Maximalspannungen in dem Spannelement verzweigt bzw. verteilt und folglich ausgeglichen werden. Hierdurch kann eine homogenere Spannungsverteilung über das Spannelement hinweg erreicht werden, wodurch Schwachstellen vermieden werden. Einem durch die Maximalspannungen auftretenden Materialverschleiß wird so besonders gut entgegengewirkt.

Das ausgebildete Gelenk verändert besonders vorteilhaft den ursprünglichen Kräfte- bzw. Spannungsverlauf, gemeint ist der Verlauf ohne Gelenk, und zwar in Bereiche mit geringeren Spannungen, wodurch das Spannelement gleichmäßig beansprucht und in manchen Bereichen sogar entlastet wird.

Mit einem mit Gelenke versehenen Spannelement kann erfindungsgemäß auch vermieden werden, dass das Spannelement im Bereich der ansonst auftretenden Maximalspannungen während der Heilung der Knochenfraktur aufgrund von überhöhten Materialbelastungen bricht und somit versagt.

Die Gelenke können darüber hinaus in Abhängigkeit der Größe des Spannelements sowie der zu behandelnden Knochenfraktur, beispielsweise Trümmerfraktur, individuell an die für die Kompression benötigten Kräfte und der daraus resultierenden Spannungen angepasst werden.

Ein weiterer Vorteil ist darin zu sehen, dass die Bruchgefahr des Spannelements bei versehentlicher Überbelastung, wie beispielsweise beim Fixieren des Spannelements an den Bruchenden der Knochen, reduziert wird. Demzufolge kann ein etwaiger Bruch des Spannelements gänzlich vermieden werden und/oder es wird der Materialermüdung des Spannelements vorgebeugt wird.

Weitere Ausgestaltungen des erfindungsgemäßen Spannelements sind Gegenstand der abhängigen Ansprüche 2 bis 8.

Das Gelenk des erfindungsgemäßen Spannelements kann verformbar sein. Mittels der Verformung des Gelenks können die Spannungen, insbesondere Maximalspannungen, vorteilhaft in dem Spannelement verteilt und ausgeglichen werden. Das Gelenk kann sich hierbei je nach aufzunehmenden Maximalspannungen entweder elastisch oder plastisch verformen. Da die Gelenke an solchen Bereichen ausgebildet sind, die weder die Positionierung noch die angestrebte Kompression stören, wirkt sich das verformbare Gelenk nicht nachteilig auf das Spannelement sowie dessen Funktionen aus.

Alternativ kann das Gelenk hingegen auch verschwenkbar sein. Durch Ausbilden eines verschwenkbaren Gelenks werden die Maximalspannungen wirkungsvoll damit aufgenommen und anschließend verteilt. Auch durch die verschwenkbare Ausbildung des Gelenks wird weder die gewünschte Kompression der Bruchenden noch die Positionierung des Spannelements beeinträchtigt.

Ferner kann das Gelenk so konfiguriert sein, dass es an Ankoppelstellen der Beugeabschnitte an die Aufnahmeabschnitte angeordnet ist. Insbesondere in diesem Bereich, da dort die Spannungen ineinander übergehen und sich addieren, werden die Maximalspannungen auf vorteilhafte Weise verzweigt und somit ausgeglichen.

Darüber hinaus kann das Gelenk des erfindungsgemäßen Spannelements in die Seitenflanken integriert sein. Da vor dem Positionieren des Spannelements selbiges, um die Längskompression der Bruchenden anschließend zu erreichen, zusammengedrückt wird, entstehen gerade in den Seitenflanken erhöhte Spannungen. Diese erhöhten Spannungen können durch Ausbilden von Gelenke in den Seitenflanken demzufolge besonders gut verteilt und ausgeglichen werden.

Zudem können an zwei gegenüberliegenden Wandungsabschnitten aufeinander zuweisende Begrenzungsstege ausgebildet sein, deren Enden sich bei einer Verformung des Spannelements berühren. Die Begrenzung eines aufgrund äußerer Kräfte eingeleiteten Verformungswegs bietet einen Schutz gegen Verformungen, die über den elastischen Bereich des Spannelements hinausgehen und zu bleibenden Veränderungen der Ausgangsmaße des Spannelements führen würden. Zudem kann der Begrenzungsanschlag als Indikator einer definierten Vorspannung des Spannelements genutzt werden.

Das Gelenk des erfindungsgemäßen Spannelements kann ein Scharnier sein. Die Integration von Scharnieren in die umlaufende Wandung kann besonders einfach erfolgen. Mit der Ausbildung eines Scharniers in der umlaufenden Wandung bedeutet dies jedoch nicht, dass die Wandung unterbrochen ist. Unter dem Begriff Scharnier kann hier ein Stangenscharnier, Federscharnier, Drehmomentscharnier, Rastscharnier, Folienscharnier, Filmscharnier oder dergleichen verstanden werden.

Das Gelenk des erfindungsgemäßen Spannelements kann hingegen auch ein Kugelgelenk sein. Gerade bei komplizierten Trümmerfrakturen ermöglicht das Kugelgelenk besonders gut die Spannungsverteilung. Auch bei einer bezüglich der Längsachse ausgebildeten Krümmung des Spannelements kann das Kugelgelenk die Maximalspannungen vorteilhaft verteilen sowie diese verringern.

Alternativ kann das Gelenk des erfindungsgemäßen Spannelements auch ein Knotenpunktgelenk sein. Das Knotenpunktgelenk verteilt an dessen Knoten besonders gut die auftretenden Spannungen, insbesondere die Maximalspannungen, und entlastet somit das Spannelement selbst.

Die vorstehend beschriebenen Merkmale und Funktionen der vorliegenden Erfindung sowie weitere Aspekte und Merkmale werden nachfolgend anhand einer detaillierten Beschreibung von bevorzugten Ausführungsformen unter Bezugnahme auf die beigefügten Figuren weiter beschrieben. Hierbei zeigt:
- Fig. 1: eine perspektivische Ansicht einer ersten Ausführungsform eines erfindungsgemäßen Spannelements mit Scharnieren;
- Fig. 2: eine Draufsicht der ersten Ausführungsform des erfindungsgemäßen Spannelements mit Scharnieren gemäß Fig.1;
- Fig. 3: eine Seitenansicht der ersten Ausführungsform des erfindungsgemäßen Spannelements mit Scharnieren gemäß Fig.1;
- Fig. 4: eine perspektivische Ansicht einer zweiten Ausführungsform eines erfindungsgemäßen Spannelements mit Knotenpunktgelenken;
- Fig. 5: eine Draufsicht der zweiten Ausführungsform des erfindungsgemäßen Spannelements mit Knotenpunktgelenken gemäß Fig.4; und
- Fig. 6: eine Draufsicht einer dritten Ausführungsform eines erfindungsgemäßen Spannelements mit Knotenpunktgelenken.

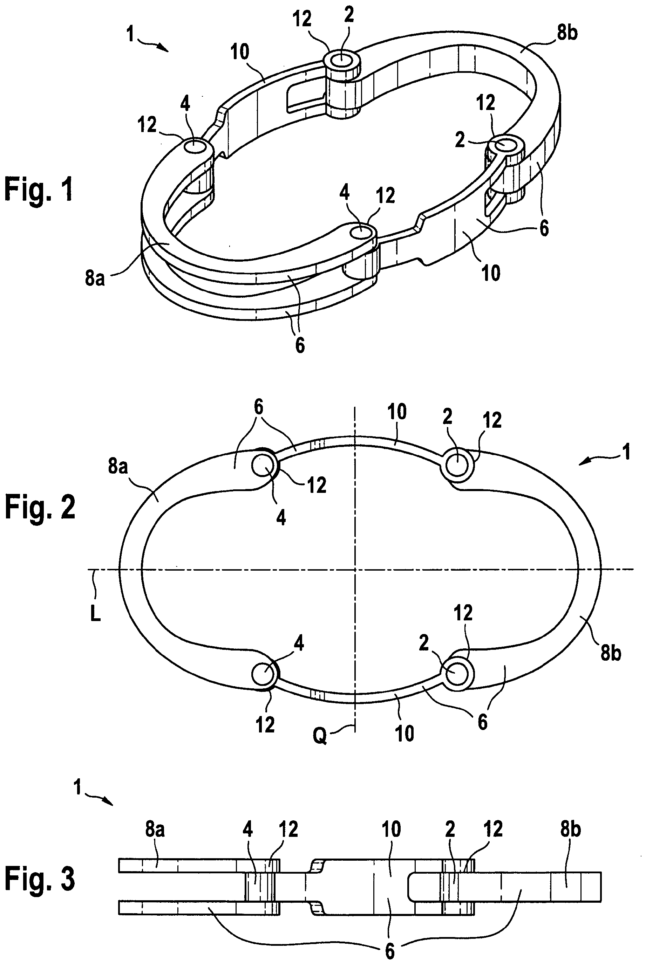

Fig. 1 stellt eine perspektivische Ansicht, Fig. 2 stellt eine Draufsicht und Fig. 3 stellt eine Seitenansicht einer ersten Ausführungsform eines erfindungsgemäßen Spannelements 1 mit Scharnieren 2, 4 dar.

Das Spannelement 1 besteht aus einem in Draufsicht hohlen, eine umlaufende Wandung 6 aufweisenden Konturkörper. Der Konturkörper weist zwei bezüglich der Querachse Q des Spannelements 1 einander gegenüberliegende Aufnahmeabschnitte 8a, 8b auf, die jeweils nach außen gekrümmt sind. Ferner weist der Konturkörper zwei bezüglich der Längsachse L des Spannelements 1 einander gegenüberliegende Seitenflanken mit Beugeabschnitten 10 auf, die gleichfalls nach außen gekrümmt sind. Das Spannelement 1 ist symmetrisch zu dessen Längsachse L konfiguriert.

Die in Fig. 3 besonders gut erkennbare korrespondierende Nut- und Federgestaltung die beiden Aufnahmeabschnitte 8a und 8b ermöglicht es, dass mehrere Spannelemente 1 miteinander verbunden werden können. So kann beispielsweise der Aufnahmeabschnitt 8b des Spannelements 1 in einem Aufnahmeabschnitt 8a eines anderen Spannelements derart eingeführt werden, dass beide Aufnahmeabschnitte fluchten. In den fluchtenden Bereich kann ein Befestigungsmittel hindurchgesteckt werden und im Anschluss daran in den Knochen eingebracht werden.

Zwischen den Beugeabschnitten 10 und den Aufnahmeabschnitten 8a, 8b befinden sich Ankoppelstellen 12, bei denen die Scharniere 2 und 4 als Gelenke vorgesehen sind. Die Scharniere 2 und 4 sind dabei verschwenkbar ausgebildet. Der Aufnahmeabschnitt 8a besteht aus zwei Teilen, die mittels der zwei Scharniere 4 miteinander verbunden sind.

Der Einsatz des Spannelements 1 zur Fixierung einer Bruchstelle kann insbesondere wie nachstehend beschrieben erfolgen.

Die Beugeabschnitte 10 des Spannelements 1 werden von Außen zusammen gedrückt, wodurch das Spannelement 1 eine Querkontraktion erfährt. Dabei tritt eine Längsdehnung des Spannelements 1 auf, d.h. der Abstand zwischen den Aufnahmeabschnitten 8a, 8b wird größer. Anschließend werden zwei nicht gezeigte Befestigungsmittel, beispielsweise Corticalisschrauben, hindurch gesteckt, so dass diese bei dem vergrößerten Abstand der Aufnahmeabschnitte 8a, 8b an dessen Innenflächen anliegen. In dieser Position werden die Befestigungsmittel in gegenüberliegende Bruchenden eines Knochens eingebracht. Nach Wegnahme der äußeren Kraft wird das Spannelement 1 aufgrund der Befestigungsmittel in der längsgedehnten Form, d.h. unter Vorspannung, gehalten. Diese Vorspannung übt dauerhaft eine Zugkraft zwischen den Befestigungsmitteln aus. Die Zugkraft zwischen den Befestigungsmitteln wird bei der Fixierungsvorrichtung zur Adaption der Bruchenden an einer Knochenfraktur angewendet.

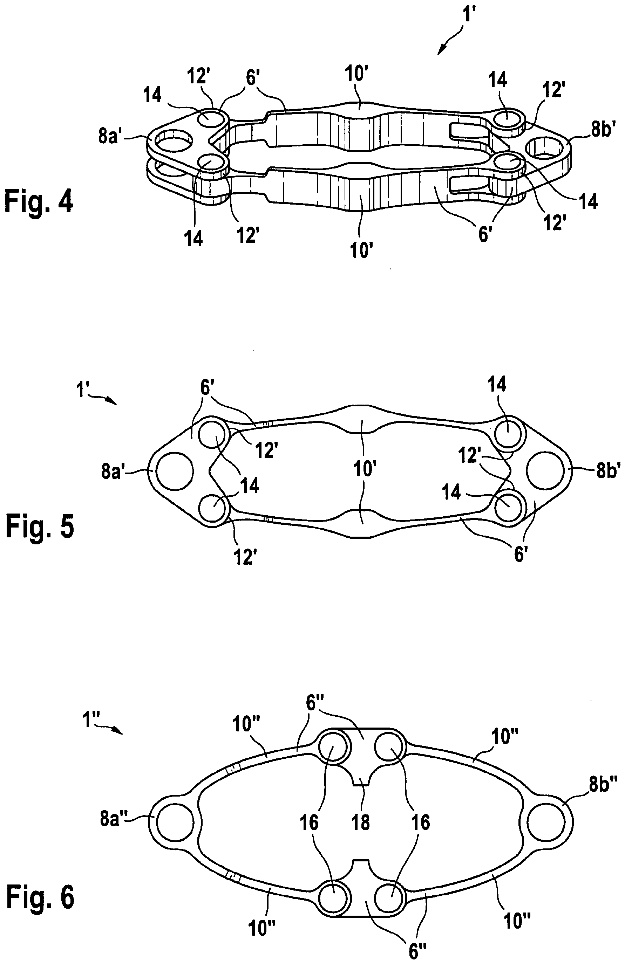

Fig. 4 stellt eine perspektivische Ansicht und Fig. 5 stellt eine Draufsicht einer zweiten Ausführungsform eines erfindungsgemäßen Spannelements 1' mit Knotenpunktgelenken dar.

Das Spannelement 1' besteht aus einem in Draufsicht hohlen, eine umlaufende Wandung 6' aufweisenden Konturkörper. Der Konturkörper weist zwei einander gegenüberliegende Aufnahmeabschnitte 8a', 8b' auf, die jeweils nach außen gekrümmt sind. Ferner weist der Konturkörper zwei einander gegenüberliegende Seitenflanken mit Beugeabschnitten 10' auf, die leicht nach außen gekrümmt sind. Anstatt der Scharniere 2, 4, wie bei der ersten Ausführungsform, sind bei den Ankoppelstellen 12' Knotenpunktgelenke 14 ausgebildet. Die Knotenpunktgelenke 14 sind dabei so ausgestaltet, dass sie sich entweder verformen oder verschwenken lassen.

Fig. 6 stellt eine Draufsicht einer dritten Ausführungsform eines erfindungsgemäßen Spannelements 1" mit Knotenpunktgelenken 16 dar. Das Spannelement 1" besteht aus einem in Draufsicht hohlen, eine umlaufende Wandung 6" aufweisenden Konturkörper. Der Konturkörper weist zwei einander gegenüberliegende Aufnahmeabschnitte 8a", 8b" auf, die jeweils nach außen gekrümmt sind. Ferner weist der Konturkörper zwei einander gegenüberliegende Seitenflanken mit Beugeabschnitten 10" auf, die auch jeweils nach außen gekrümmt sind. In den beiden Seitenflanken sind jeweils zwei Knotenpunktgelenke 16 ausgebildet, um die in den Beugeabschnitten 10" herrschenden Maximalspannungen abzubauen. Darüber hinaus sind bei jeder Seitenflanke mittig zwischen den beiden Knotenpunktgelenken 16 ein Begrenzungssteg 18 ausgebildet, so dass die Beugeabschnitte 10" lediglich bis zu einem vorbestimmten Maße zusammengedrückt werden können.

Das Spannelement 1, 1',1" wird aus biokompatiblen Materialen hergestellt. Zudem ist ebenfalls ein Hybridaufbau des Spannelements 1, 1',1"möglich, bei dem die Wandung der Beugeabschnitte 8 aus einem Material mit der gewünschten elastischen Eigenschaft bestehen und weitere Bereiche des Spannelements 1, 1',1", wie z.B. die Aufnahmeabschnitte 6a, 6b, aus einem steifen oder resorbierbaren Material gebildet werden.

Als Ausgangsmaterial für das Spannelement 1, 1',1" kommen beispielsweise Metalle aus der Gruppe: X42CrMo15, X100CrMo17, X2CrNiMnMoNNb21-16-5-3, X20Cr13, X15Cr13, X30Cr13, X46Cr13, X17CrNi16-2, X14CrMoS17, X30CrMoN15-1, X65CrMo 17-3, X55CrMo14, X90CrMoV18, X50CrMoV15, X 38CrMo V15, G-X 20CrMo13, X39CrMo17-1, X40CrMoVN16-2, X105CrMo17, X20CrNiMoS13-1, X5CrNi18- 0, X8CrNiS18-9, X2CrNi19-11, X2CrNi18-9, X10CrNi18-8 X5CrNiMo17-12-2, X2CrNiMo17-12-2, X2CrNiMoN25-7-4, X2CrNiMoN17-13-3, X2CrNiMo17-12-3, X2CrNiMo18-14-3, X2CrNiMo18-15-3; X 2 CrNiMo 18 14 3, X13CrMnMoN18-14-3, X2CrNiMoN22136, X2CrNiMnMoNbN21-9-4-3, X4CrNiMnMo21-9-4, X105CrCoMo18-2, X6CrNiTi18-10, X5CrNiCuNb16-4, X3CrNiCuTiNb12-9, X3CrNiCuTiNb12-9, X7CrNiA117-7, CoCr20Ni15Mo, G-CoCr29Mo, CoCr20W15Ni, Co-20Cr-15W-10Ni, CoCr28MoNi, CoNi35Cr20Mo10, Ti1, Ti2, Ti3, Ti4, Ti-5Al-2,5Fe, Ti-5Al-2,5Sn, Ti-6Al-4V, Ti-6Al-4V ELI, Ti-3Al-2,5V (Gr9), 99,5Ti, Ti-12Mo-6Zr-2Fe, Ti-13,4Al-29Nb, Ti-13Nb-13Zr, Ti-15Al, Ti-15Mo, Ti- 15Mo-5Zr-3Al, Ti-15Sn, Ti-15Zr-4Nb, Ti-15Zr-4Nb-4Ta, Ti-15Zr-4Nb-4Ta-0,2Pd, Ti-29Nb-13Ta-4,6Zr, Ti-30Nb-10Ta-5Zr, Ti-35,5Nb-1,5Ta-7,1Zr, Ti-35Zr-10Nb, Ti-45Nb, Ti-30Nb, Ti-30Ta, Ti-6Mn, Ti-5Zr-3Sn-5Mo-15Nb, Ti-3Al-8V-6Cr-4Zr-4Mo, Ti-6Al-2Nb-1Ta-0,8Mo, Ti-6Al-4Fe, Ti-6Al-4Nb, Ti-6Al-6Nb-1Ta, Ti-6A1-7Nb, Ti-6Al-4Zr-2Sn-2Mo, Ti-8,4Al-15,4Nb, Ti-8Al-7Nb, Ti-8Al-1Mo-1V, Ti-11Mo-6Zr-4Sn, in Betracht.

Ferner kommen Polymere aus der Gruppe: MBS, PMMI, MABS, CA, CTA, CAB, CAP, COC, PCT, PCTA, PCTG, EVA, EVAL, PTFE, ePTFE, PCTFE, PVDF, PVF, ETFE, ECTFE, FEP, PFA, LCP, PMMA, PMP, PHEMA, Polyamid 66, Polyamid 6, Polyamid 11 , Polyamid 2, PAEK, PEEK, PB, PC, PPC, PETP, PBT, MDPE, LDPE, HDPE, UHMWPE, LLDPE, PI, PAI, PEI, PIB, POM, PPO, PPE, PPS, PP, PS, PSU, PESU, PVC, PVC-P, PVC-U, ABS, SAN, TPE-U, TPE-A, TPE-E, PVDC, PVA, SI, PDMS, EPM, EP, UF, MF, PF, PUR, UP, PEBA, PHB, PLA, PLLA, PDLA, PDLLA, PGL, PGLA, PGLLA, PGDLLA, PGL-co-poly TMC, PGL-co-PCL, PDS, PVAL, PCL, Poly-TMC, PUR (linear), NiTi Superelastic, NiTi Shape Memory, in Betracht.

Des Weiteren kommen Keramiken aus der Gruppe: Al₂O₃ (Aluminiumoxid), Y-TZP (Zirkonoxidkeramik), AMC (Alumina Matrix Composite), HA (Hydroxilapatit), TCP (Tricalciumphosphat), Ceravital (Glaskeramik/Bioglas®), FZM/K (Zirkonoxid, teilstabilisiert), TZP-A (Zirkonoxidkeramik), ATZ (Alumina-toughened Zirconia), C799 (Aluminiumoxidkeramik), Schott 8625 (Transponderglas), in Betracht.

Darüber hinaus kommen Kombinationen hiervon in Betracht.

Die Erfindung lässt neben den erläuterten Ausführungsformen weitere Gestaltungsansätze zu.

Obgleich die zweite und die dritte Ausführungsform keine korrespondierende Nut- und Federgestaltung der beiden Aufnahmeabschnitte aufweist, können diese derart konfiguriert werden, um zu einem wie in der DE 20 2011 109808 U1 dargestellten Modularsystem erweitert zu werden.

Als weitere alternative Ausführungsform kann das Spannelement wenigstens jeweils ein Knotenpunktgelenk 16 bei den Seitenflanken und jeweils ein Knotenpunktgelenk 14 bei den Ankoppelstellen aufweisen.

Obschon die vorstehenden Ausführungsformen symmetrisch zu deren Längsache L konfiguriert sind, ist es ferner denkbar, lediglich zwei Gelenke bei draufsichtiger Betrachtung des Spannelements diametral gegenüberliegend anzuordnen.

Zwar wurde bei den vorstehenden Ausführungsformen jeweils für eine Ausführungsform nur eine Art von Gelenken ausgebildet, doch ist das Spannelement auch so konfigurierbar, dass es unterschiedliche Gelenke aufweisen kann. So können beispielsweise im stirnseitigen Aufnahmeabschnitt 8a Scharniere verwendet werden und im gegenüberliegenden stirnseitigen Aufnahmeabschnitt 8b Knotenpunktgelenke. Es ist jedoch jede andere Kombination konfigurierbar.

Obwohl vorstehend eine Seitenflanke einen Beugeabschnitt aufweist, kann die Seitenflanke auch so gestaltet werden, dass diese mehrere Beugeabschnitte aufweist.

Darüber hinaus ist zu berücksichtigen, dass die jeweiligen Aufnahmeabschnitte fließend in die Beugeabschnitte übergehen können.

## Patentansprüche

1. Spannelement (1, 1'; 1") zum Fixieren von Bruchenden von Knochen einer Knochenfraktur, aufweisend
einen in Draufsicht hohlen, eine umlaufende Wandung (6, 6'; 6") aufweisenden Konturkörper, mit
zwei bezüglich einer Querachse (Q) des Spannelements (1, 1'; 1") einander gegenüberliegenden stirnseitigen Aufnahmeabschnitten (8a, 8b; 8a', 8b'; 8a", 8b") zur Aufnahme von Befestigungsmitteln, die durch den Konturkörper hindurchsteckbar sind, und
zwei bezüglich einer Längsachse (L) des Spannelements (1, 1'; 1 ") einander gegenüberliegenden Seitenflanken mit Beugeabschnitten (10, 10'; 10"),
wobei die umlaufende Wandung (6,6'; 6") des Konturkörpers wenigstens abschnittsweise im Bereich der stirnseitigen Aufnahmeabschnitte (8a, 8b; 8a', 8b'; 8a", 8b") und im Bereich der seitlichen Beugeabschnitte (10, 10'; 10") federelastisch verformbar ist, und
wobei über die Beugeabschnitte (10, 10'; 10") ein definiertes elastisches Verhalten des Spannelements (1, 1'; 1") derart eingeprägt ist, dass eine vorbestimmte Spannung zwischen den Aufnahmeabschnitten (8a, 8b; 8a', 8b'; 8a", 8b") erzeugbar ist, zur Aufbringung einer Zugkraft zwischen den durch den Konturkörper hindurchsteckbaren Befestigungsmitteln zum Fixieren der Bruchenden der Knochen bei einer Knochenfraktur,
**dadurch gekennzeichnet, dass** sich zwischen den seitlichen Beugeabschnitten (10, 10'; 10") und den stirnseitigen Aufnahmeabschnitten (8a, 8b; 8a', 8b'; 8a", 8b") Ankoppelstellen (12) befinden, und wenigstens ein Gelenk zum Ausgleich und Verteilen von Spannungsspitzen im Bereich der umlaufenden Wandung (6, 6'; 6") an den Ankoppelstellen (12) zwischen den seitlichen Beugeabschnitten (10, 10'; 10") und den stirnseitigen Aufnahmeabschnitten (8a, 8b; 8a', 8b'; 8a", 8b") angeordnet ist.

2. Spannelement nach Anspruch 1, **dadurch gekennzeichnet, dass** das wenigstens eine Gelenk elastisch oder plastisch verformbar ist.

3. Spannelement nach Anspruch 1, **dadurch gekennzeichnet, dass** das wenigstens eine Gelenk verschwenkbar ist.

4. Spannelement nach einem der Ansprüche 1 bis 3, **dadurch gekennzeichnet, dass** das wenigstens eine Gelenk in die Seitenflanken integriert ist.

5. Spannelement nach einem der Ansprüche 1 bis 4, **dadurch gekennzeichnet, dass** an zwei gegenüberliegenden Wandungsabschnitten aufeinander zuweisende Begrenzungsstege (18) ausgebildet sind, deren Enden sich bei einer Verformung des Spannelements (1, 1'; 1") berühren.

6. Spannelement nach einem der Ansprüche 1 bis 5, **dadurch gekennzeichnet, dass** das Gelenk ein Scharnier (2, 4) ist.

7. Spannelement nach Anspruch 1 oder einem der Ansprüche 3 bis 5, **dadurch gekennzeichnet, dass** das Gelenk ein Kugelgelenk ist.

8. Spannelement nach einem der Ansprüche 1 bis 5, **dadurch gekennzeichnet, dass** das Gelenk ein Knotenpunktgelenk (14) ist.

## Claims

1. A tensioning element (1, 1'; 1") for fixing the fracture ends of bones in a bone fracture, comprising:
a contoured body which is hollow when viewed from above and comprises a circumferential wall (6, 6'; 6"), comprising
two face sided receiving sections (8a, 8b; 8a', 8b'; 8a", 8b") lying opposite to each other with respect to a lateral axis (Q) of the tensioning element (1, 1', 1"), for receiving fixing means which can be pushed through the contoured body, and
two lateral flanks lying opposite to each other with respect to a longitudinal axis (L) of the tensioning element (1, 1', 1") and having angled sections (10, 10', 10")
wherein the circumferential wall (6, 6', 6") of the contoured body is resiliently deformable at least in sections in the region of the face sided receiving sections (8a, 8b; 8a', 8b'; 8a", 8b") and in the region of the lateral angled sections (10, 10', 10"), and
wherein a defined elastic behavior of the clamping element (1, 1'; 1") can be impressed via the angled sections (10, 10', 10") in such a way that a predetermined tension can be generated between the receiving sections (8a, 8b; 8a', 8b'; 8a", 8b") for applying a tensile force between the fixing means which can be pushed through the contoured body for fixing the fracture ends in a bone fracture,
**characterized in that**
between the lateral angled sections (10, 10', 10") and the face sided receiving sections (8a, 8b; 8a', 8b'; 8a", 8b") there are provided docking points (12), and
at least one articulation for compensation and distribution of stress peaks is positioned in the region of the circumferential wall (6, 6', 6") at the docking points (12) between the lateral angled sections (10, 10', 10") and the face sided receiving sections (8a, 8b; 8a', 8b'; 8a", 8b").

2. The tensioning element according to claim 1, **characterized in that** the at least one articulation is elastically or plastically deformable.

3. The tensioning element according to claim 1, **characterized in that** the at least one articulation is rotatable.

4. The tensioning element according to anyone of claims 1 to 3, **characterized in that** the at least one articulation is integrated in the lateral flanks.

5. The tensioning element according to anyone of claims 1 to 4, **characterized in that** at two wall sections opposing each other restriction bars (18) facing each other are formed, their ends contacting each other when the tensioning element (1, 1'; 1") is deformed.

6. The tensioning element according to anyone of claims 1 to 5, **characterized in that** the articulation is a hinge (2, 4).

7. The tensioning element according to claim 1 or anyone of claims 3 to 5, **characterized in that** the articulation is a ball joint.

8. The tensioning element according to anyone of claims 1 to 5, **characterized in that** the articulation is a node joint (14).

## Revendications

1. Élément de serrage (1, 1' ; 1") pour fixer des extrémités d'os fracturées d'une fracture osseuse, présentant
un corps de contour présentant une paroi périphérique (6, 6' ; 6"), creux dans la vue de dessus avec
deux sections de réception côté frontal (8a, 8b ; 8a', 8b' ; 8a" , 8b") opposées l'une à l'autre par rapport à un axe transversal (Q) de l'élément de serrage (1, 1' ; 1") pour recevoir des moyens de fixation, qui sont enfichables par le corps de contour, et
deux flancs latéraux opposés l'un à l'autre par rapport à un axe longitudinal (L) de l'élément de serrage (1, 1' ; 1") avec des sections de flexion (10, 10' ; 10"),
dans lequel la paroi périphérique (6, 6' ; 6") du corps de contour est élastiquement déformable au moins par section dans la zone des sections de réception côté frontal (8a, 8b ; 8a', 8b' ; 8a'', 8b") et dans la zone des sections de flexion latérale (10, 10' ; 10"), et
dans lequel un comportement élastique défini de l'élément de serrage (1, 1' ; 1") est imprimé par le biais des sections de flexion (10, 10' ; 10") de telle sorte qu'un serrage prédéfini peut être généré entre les sections de réception (8a, 8b ; 8a', 8b' ; 8a'', 8b"), pour l'application d'une force de traction entre les moyens de fixation enfichables par le corps de contour pour fixer des extrémités d'os fracturées lors d'une fracture osseuse,
**caractérisé en ce que**
des points d'accouplement (12) se trouvent entre les sections de flexion latérales (10, 10' ; 10") et les sections de réception côté frontal (8a, 8b ; 8a', 8b' ; 8a" , 8b"), et
au moins une articulation pour compenser et distribuer des pics de tension est agencée dans la zone de la paroi périphérique (6, 6' ; 6") au niveau des points d'accouplement (12) entre les sections de flexion latérales (10, 10' ; 10") et les sections de réception côté frontal (8a, 8b ; 8a', 8b' ; 8a" , 8b")

2. Élément de serrage selon la revendication 1, **caractérisé en ce que** l'au moins une articulation est élastiquement ou plastiquement déformable.

3. Élément de serrage selon la revendication 1, **caractérisé en ce que** l'au moins une articulation est pivotante.

4. Élément de serrage selon l'une quelconque des revendications 1 à 3, **caractérisé en ce que** l'au moins une articulation est intégrée dans les flancs latéraux.

5. Élément de serrage selon l'une quelconque des revendications 1 à 4, **caractérisé en ce que** des nervures de limitation (18) tournées l'une vers l'autre au niveau de deux sections de paroi opposées sont réalisées, dont les extrémités se touchent lors d'une déformation de l'élément de serrage (1, 1' ; 1").

6. Élément de serrage selon l'une quelconque des revendications 1 à 5, **caractérisé en ce que** l'articulation est une charnière (2, 4).

7. Élément de serrage selon la revendication 1 ou selon l'une quelconque des revendications 3 à 5, **caractérisé en ce que** l'articulation est une articulation sphérique.

8. Élément de serrage selon l'une quelconque des revendications 1 à 5, **caractérisé en ce que** l'articulation est une articulation à point de jonction (14).
